(19) Europäisches Patentamt
European Patent Office
Office européen des brevets

(11) Publication number: **0 149 564**
**B1**

(12) # EUROPEAN PATENT SPECIFICATION

(45) Date of publication of patent specification: **06.05.87**

(51) Int. Cl.[4]: **C 07 C 67/40**, C 07 C 69/06// C07C29/136, C07C29/14

(21) Application number: **85300333.3**

(22) Date of filing: **17.01.85**

(54) **Process for the production of methyl formate.**

(30) Priority: **18.01.84 GB 8401237**

(43) Date of publication of application: **24.07.85 Bulletin 85/30**

(45) Publication of the grant of the patent: **06.05.87 Bulletin 87/19**

(84) Designated contracting states: **BE DE FR GB IT NL**

(56) References cited: **None**

(73) Proprietor: **BP Chemicals Limited**
**Belgrave House 76 Buckingham Palace Road**
**London, SW1W 0SU (GB)**

**The University of Sheffield**
**Sheffield S3 7HF (GB)**

(72) Inventor: **Maitlis, Peter Michael**
**University of Sheffield**
**Sheffield, S3 7HF (GB)**
Inventor: **Smith, Thomas Ashley**
**55 Bakewell Road, Hazel Grove**
**Stockport, Cheshire, SK7 6JT (GB)**

(74) Representative: **Fawcett, Richard Fennelly**
**BP INTERNATIONAL LIMITED Patents Division Chertsey Road**
**Sunbury-on-Thames Middlesex, TW16 7LN (GB)**

**EP 0 149 564 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to a process for the conversion of methanol to methyl formate, more particularly to a process operated in the gas or liquid phase using a homogeneous platinum group metal catalyst.

Methyl formate is a useful organic chemical and can be used as an intermediate for the preparation of a wide range of other chemicals including formic acid itself and higher carboxylic acids such as acetic and propionic acid and their esters.

The preparation of methyl formate from methanol is a commercially attractive proposition. Thus methanol is readily available commodity chemical obtained from a variety of cheap hydrocarbon feedstocks, e. g. natural gas and synthesis gas. Methyl formate has been prepared previously by reaction of methanol and carbon monoxide and by reaction of methanol and carbon dioxide using Group VIII metal catalysts.

A possible method of preparing methyl formate from methanol, without the need for coreactants, exists namely the reaction

$$2CH_3OH \rightarrow CH_3O_2CH + H_2$$

in which a molecule of methanol is oxidatively dehydrogenated. In order to carry this reaction out it is necessary to have, in addition to methanol and a catalyst, a feedstock capable of accepting the hydrogen produced during the reaction. This hydrogen acceptor feedstock is conveniently an organic molecule capable of undergoing a hydrogenation reaction with the hydrogen, for example an aldehyde, ketone or an olefin.

J. Organic Chemistry (1974) Volume 39 page 1622 discloses a process in which alcohols are oxidatively dehydrogenated to the corresponding aldehyde or ketone in the presence of $RhH(PPh_3)_4$ (Ph = phenyl) and, as hydrogen acceptor a cycloolefin. In this reference however all the alcohols were oxidatively dehydrogenated during the reaction with the exception of methanol which appeared substantially unreactive.

It has now been found that methanol can be converted to methyl formate using a wide range of hydrogen acceptors and using a platinum group metal catalyst.

Accordingly, the present invention provides a process for the preparation of methyl formate characterised in that methanol is reacted with a hydrogen acceptor at elevated temperature in the presence of a platinum group metal catalyst.

As mentioned previously, the hydrogen acceptor is suitably an organic molecule capable of undergoing a hydrogenation reaction with the hydrogen denated by the methanol and includes species such as aldehydes, ketones and olefins. In the case of an aldehyde or a ketone, the hydrogenation product is the corresponding alcohol, while in the case of an olefin hydrogenation of the double bond occurs.

Any aldehyde, ketone or olefin can be used in principle although it is preferable to choose a hydrogen acceptor whose hydrogenation product can be easily separated from the other reaction components. Suitably $C_1$-$C_{20}$, preferably $C_1$-$C_{10}$, aldehydes, ketones or olefins are used. Preferred hydrogen acceptors include, for example, cyclohexanone, methyl ethyl ketone, acetone and acetaldehyde.

The conversion of methanol to methyl formate in the presence of a hydrogen acceptor is catalysed by a platinum group metal catalyst. By platinum group metal is meant a metal or metals selected from the group consisting of ruthenium, rhodium palladium, osmium, iridium and platinum. Preferably the platinum group metal is ruthenium.

The platinum group metal catalyst can be a simple salt or an inorganic or organometallic complex of the platinum group metal. Such complexes may contain ligands such a halide ions, amines, phosphines, arsines and stibines. In addition ligands able to Π-bond to the metal, e. g. the cyclopentadienyl and pentamethyl cyclopentadienyl anions and benzene and its substituted derivatives can also be present. The complex of the platinum group metal can be cationic, anionic or neutral and can be prepared separately or generated in situ in the reaction mixture.

In a preferred form, the catalyst is a phosphine or phosphite complex of the platinum group metal which can be formed in situ by reaction of a metal complex with a phosphine or phosphite. In this case the preferred platinum group metals are ruthenium.

The phosphine or phosphite used can be a monodentate, bidentate or polydentate phosphine or phosphite. Preferred phosphines are trialkylphosphines e. g. tributylphosphine tridrylphosphines e. g. triphenylphosphine or phosphines having both alkyl or aryl groups attached to the phosphorus atom e. g. diphos, dppe and the like. Preferred phosphites are trialkylphosphites and triarylphosphites.

When salts and complexes of the type described above are used it is preferable to operate the process as a liquid phase process with the catalyst soluble or partially soluble in the reaction mixture.

As an alternative to a liquid phase.process the reaction may be effected in the gaseous or vapour phase. In this mode of operation, the hydrogen acceptor, together with the methanol, are vapourised and passed over or through a bed of solid catalyst. Suitably, the solid catalyst comprises the platinium group metal supported on an inert carrier. The carrier can be inorganic, for example ametaloxide, silica. alumina, an aluminosilicate, clay or diatomaceous earth or organic for example polymers, such as

polystyrene, polystyrene/divinylbenzene copolymers and fluorinated polymers.

Various methods of supporting the platinum group metal, on the inert carrier are available and are familiar to the skilled man. Examples include impregnation, ion-exchange and coprecipitation methods. A preferred method involves chemically binding a ligand to the inert carrier, for example a phosphine ligand, and then complexing the bound phosphine ligand with a platinum group compound. The phosphine is bound to the carrier by means of one or more reactive groups attached to the phosphine.

The solid catalyst can, of course also be used in association with a liquid phase mode of operation, although in this case the catalyst will be insoluble in the reaction medium. However, for both modes of operation it is preferable that the molar ratio of methanol to hydrogen acceptor is greater than 1 : 1 preferably 10 : 1.

The reaction is suitably carried out at elevated temperature in the range 100-240 °C although higher temperatures may be used if desired. Preferably the reaction temperature should be in the range 140-185 °C.

The reaction may be carried out at pressures up to 20 bars, preferably in the range 5-10 bars. When the reaction is carried out at superatmospheric pressure, the pressure may be generated by the autogenous pressure of one or more of the reactants at the temperature of reaction, or it may be generated by applying an overpressure of a suitable inert gas e. g. nitrogen, or a combination of both.

The process as described can be carried out either batchwise or continuously. When the process is operated in the gas phase, continuous operation is preferred.

The invention is illustrated by the following examples.

Examples 1 to 8

Cyclohexanone (8 mmol) and methanol (195 mmol) and catalyst (0.08 mmol) were heated to 150 °C in a Fisher-Porter tube. At the end of the reaction time recorded in Table 1 below, the contents were cooled and analysed by gas chromatography.

In Examples 6-8 the ruthenium trichloride used was ruthenium chloride hydrate supplied by Johnson-Matthey. The catalysts therefore refer to one mole of ruthenium chloride hydrate and the appropriate number of moles of triphenylphosphine.

Examples 1 to 8 illustrate the following reaction using different catalysts and varying reaction times.

Cyclohexanone + methanol → cyclohexanol + methyl formate.

Table 1

| Example | Catalyst | Reaction time (hours) | Cyclohexanol mmol and moles of product per mole of catalyst | Methyl formate mmol and moles of product per mole of catalyst |
|---|---|---|---|---|
| 1 | $[(C_5Me_5Rh)_2Cl_4]$ | 19 | 1.1 (14) | 1.3 (16) |
| 2 | $[(C_5Me_5Ir)_2Cl_4]$ | 18 | 1.2 (15) | trace. |
| 3 | $[(C_6Me_6Ru)_2Cl_4]$ | 18 | 0.5 (6) | trace. |
| 4 | $(Ph_3P)_3RuCl_2$ | 9.5 | 6.8 (85) | 4.8 (60) |
| 5 | $(Ph_3P)_3OsHBr(CO)$ | 18 | 1.0 (12) | 2.8 (35) |
| 6 | $RuCl_3$+1molePPh$_3$ | 18 | 0.3 (3) | 3.3 (42) |
| 7 | $RuCl_3$+2molesPPh$_3$ | 18 | 0.3 (4) | 3.6 (45) |
| 8 | $RuCl_3$+3molesPPh$_3$ | 18 | 6.2 (77) | 3.5 (44) |

(See Table 2 page 4)

Table 2

| Example | Hydrogen Acceptor | Product | mmol of product and moles/mole catalyst | Methyl formate mmol and moles/mole catalyst |
|---|---|---|---|---|
| 9 | cyclohexanone | cyclohexanol | 5.4 (67) | 2.0 (25) |
| 10 | PhCOMe | PhCHOHMe | 0.4 (5) | 2.1 (26) |
| 11 | MeCOEt | MeCHOHEt | 1.5 (19) | 1.5 (18) |
| 12 | PhCHO | $PhCH_2OH$ | 1.4 (17) | trace. |
| 13 | 4t-butyl-cyclohexanone | 4-t butyl-cyclohexanol (trans:cis=4:1) | 6.2 (77) | 0.7 (9) |
| 14 | $Me_2C=CHCOMe$ | $Me_2CHCH_2COMe$ | 7.4 (92) | 2.8 (35) |
| 15 | cyclohexene | cyclohexane | 0.2 (3) | trace. |

Examples 9 to 15

These examples illustrate the reaction of methanol as a hydrogen donor with various hydrogen acceptors using, as catalyst, $(Ph_3P)_3RuCl_2$ which was the most active catalyst recorded in Table 1. The reaction may be summarised :

methanol + hydrogen acceptor → product + methyl formate.

In the examples the hydrogen acceptor (8 mmol), methanol (200 mmol) and $(Ph_3P)_3RuCl_2$ (0.08 mmol) were heated in a Fisher-Porter tube at 139 to 148 °C for 18 hours. At the end of the reaction time the contents were cooled and analysed by gas chromatography.

Example 16

Cyclohexanone (4.9 $cm^3$, 48 mmol) methanol (55 $cm^3$) and $[(C_5Me_5Rh)_2(OH)_3]Cl$ (0.3 g, 0.5 mmol) were heated in a stainless steel autoclave under nitrogen for 5 hours at 150 °C and 60 bar. Analysis showed the presence of cyclohexanol (4.1 mmol ; 8 mol/mol catalyst) and methyl formate (2.1 mmol ; 4 mol/mol catalyst).

Example 17

Methylvinylketone (3.9 $cm^3$, 48 mmol) methanol (56 $cm^3$) and $[(C_5Me_5Rh)_2(OH)_3]Cl$ (0.3 g, 0.5 mmol) were heated in a stainless steel autoclave under nitrogen for 5 hours at 150 °C and 60 bar. Analysis showed the presence of methylethylketone (butan-2-one) (25 mmol ; 52 mol/mol) catalyst and methyl formate (12 mmol ; 25 mol/mol catalyst).

Example 18

Example 18 illustrates that the process of the present invention may be carried out in the gas phase using a platinum group metal catalyst supported on an inert carrier.

(a) Preparation of the Catalyst

Davison 57 grade silica gel (mesh 30-60) was functionalised by reaction with the phosphine $(EtO)_3SiCH_2CH_2PPh_2$). The functionalised silica (10 g. 0.8 % wt P) was treated with $RuCl_3$ in 200 mls of refluxing ethanol. After ca 2 hours the mixture was cooled, the solid separated by filtration and the remaining solvent removed under vacuum. Care was later to exclude air all times during the preparation. The resulting catalyst was bottle-green.

(b) Gas phase process

4 g of the catalyst described in (a) was charged to a tubular glass reactor (0.5 cm id) contained in an oven and equipped with preheat section, liquid feed pump, gas feed system and product recovery section. Nitrogen (30 ml/min) and a 1 : 1 mixture of methanol and acetone (liquid feed equivalent to 15 ml gas/min) were then passed over the catalyst. During this time the catalyst was maintained at 180 °C. After ca 4

hours the feed was stopped and the recovered product analysed. The product contained 0.3 % wt methyl formate and 0.9 % wt % isopropanol as determined by G. C.

## Claims

1. A process for the preparation of methyl formate characterised in that methanol is reacted with a hydrogen acceptor at elevated temperature in the presence of a platinum group metal.

2. A process as claimed in claim 1, characterised in that the hydrogen acceptor is selected from the group consisting of aldehydes, ketones and olefins.

3. A process as claimed in claim 2 characterised in that the reaction is carried out in the liquid phase using a platinum group metal catalyst which is soluble in the reaction mixture.

4. A process as claimed in claim 3 characterised in that the platinum group metal catalyst is a simple salt, or an inorganic or organometallic complex of a metal selected from the group consisting of ruthenium, rhodium, palladium, osmium, iridium and platinum.

5. A process as claimed in claim 4 characterised in that the platinum group metal catalyst is a phosphine or phosphite complex of ruthenium.

6. A process as claimed in claim 5 characterised in that the phosphine or phosphite complex of ruthenium contains at least one ligand selected from the group which consists of trialkylphosphines, triarylphosphines, trialkylphosphites and triarylphosphites.

7. A process as claimed in claim 5 characterised in that the phosphine or phosphite complex is formed in situ from a ruthenium compound and a phosphine or phosphite.

8. A process as claimed in claim 1 characterised in that the reaction is carried out in the vapour phase in the presence of a bed of the platinum group catalyst.

9. A process as claimed in claim 8 characterised in that the platinum group metal catalyst comprises a platinum group metal compound supported on an inert carrier.

10. A process as claimed in claim 9 characterised in that (i) the inert carrier is functionalised by chemically bonding a ligand to the inert carrier and (ii) a platinum group metal compound is subsequently complexed to the ligand.

11. A process as claimed in claim 8 characterised in that the platinum group metal catalyst is a ruthenium catalyst.

## Patentansprüche

1. Verfahren zur Herstellung von Methylformiat, dadurch gekennzeichnet, daß Methanol mit einem Wasserstoff-Akzeptor bei erhöhter Temperatur in Gegenwart eines Metalls der Platingruppe umgesetzt wird.

2. Verfahren nach Anspruch 1, dadurch gekennzeichnet, daß der Wasserstoff-Akzeptor ausgewählt wird aus der Gruppe, die besteht aus Aldehyden, Ketonen und Olefinen.

3. Verfahren nach Anspruch 2, dadurch gekennzeichnet, daß die Umsetzung in der flüssigen Phase unter Verwendung eines Katalysators der Platinmetall-Gruppe, der in der Reaktionsmischung löslich ist, durchgeführt wird.

4. Verfahren nach Anspruch 3, dadurch gekennzeichnet, daß der Katalysator der Platinmetall-Gruppe ein einfaches Salz oder ein anorganischer oder organometallischer Komplex eines Metalls, ausgewählt aus der aus Ruthenium, Rhodium, Palladium, Osmium, Iridium und Platin bestehenden Gruppe ist.

5. Verfahren nach Anspruch 4, dadurch gekennzeichnet, daß der Katalysator der Platinmetall-Gruppe ein Phosphin- oder Phosphitkomplex von Ruthenium ist.

6. Verfahren nach Anspruch 5, dadurch gekennzeichnet, daß der Phosphin- oder Phosphitkomplex von Ruthenium mindestens einen Liganden enthält, ausgewählt aus der Gruppe, die besteht aus Trialkylphosphinen, Triarylphosphinen, Trialkylphosphiten und Triarylphosphiten.

7. Verfahren nach Anspruch 5, dadurch gekennzeichnet, daß der Phosphin- oder Phosphitkomplex in situ aus einer Ruthenium-Verbindung und einem Phosphin oder Phosphit gebildet wird.

8. Verfahren nach Anspruch 1, dadurch gekennzeichnet, daß die Umsetzung in der Dampfphase in Gegenwart eines Bettes des Katalysators der Platingruppe durchgeführt wird.

9. Verfahren nach Anspruch 8, dadurch gekennzeichnet, daß der Katalysator der Platinmetall-Gruppe eine Verbindung der Platinmetall-Gruppe, getragen auf einem inerten Träger, umfaßt.

10. Verfahren nach Anspruch 9, dadurch gekennzeichnet, daß (i) der inerte Träger durch chemisches Binden eines Liganden an den inerten Träger funktionstüchtig gemacht wird und (ii) eine Verbindung der Platinmetall-Gruppe anschließend an den Liganden komplexiert wird.

11. Verfahren nach Anspruch 8, dadurch gekennzeichnet, daß der Katalysator der Platinmetall-Gruppe ein Rutheniumkatalysator ist.

**Revendications**

1. Procédé pour la préparation du formiate de méthyle, caractérisé en ce qu'on fait réagir le méthanol avec un accepteur d'hydrogène à température élevée en présence d'un métal du groupe du platine.

2. Procédé selon la revendication 1, caractérisé en ce que l'accepteur d'hydrogène est choisi parmi le groupe consistant en des aldéhydes, des cétones et des oléfines.

3. Procédé selon la revendication 2, caractérisé en ce qu'on effectue la réaction en phase liquide, en utilisant un catalyseur, à base d'un métal du groupe du platine, qui est soluble dans le mélange réactionnel.

4. Procédé selon la revendication 3, caractérisé en ce que le catalyseur à base d'un métal du groupe du platine est un sel simple, ou est un complexe minéral ou organo-métallique d'un métal choisi parmi le groupe consistant en le ruthénium, le rhodium, le palladium, l'osmium, l'irridium et le platine.

5. Procédé selon la revendication 4, caractérisé en ce que le catalyseur à base d'un métal du groupe du platine est un complexe de phosphine ou de phosphite de ruthénium.

6. Procédé selon la revendication 5, caractérisé en ce que le complexe de phosphine ou de phosphite de ruthénium contient au moins un ligand choisi parmi le groupe qui consiste en des trialkylphosphines, des triarylphosphines, des phosphites de trialkyle et des phosphites de triaryle.

7. Procédé selon la revendication 5, caractérisé en ce que le complexe de phosphine ou phosphite est formé in situ (sur place) à partir d'un composé de ruthénium et d'une phosphine ou d'un phosphite.

8. Procédé selon la revendication 1, caractérisé en ce qu'on effectue la réaction en phase vapeur en présence d'un lit de catalyseur à base d'un métal du groupe du platine.

9. Procédé selon la revendication 8, caractérisé en ce que la catalyseur à base d'un métal du groupe du platine comprend un composé d'un métal du groupe du platine supporté sur un support inerte.

10. Procédé selon la revendication 9, caractérisé en ce que (i) on confère au support inerte une ou des fonctions chimiques en liant chimiquement un ligand au support inerte, et (ii) on complexe ensuite sur le ligand un composé de métal du groupe du platine.

11. Procédé selon la revendication 8, caractérisé en ce que le catalyseur à base d'un métal du groupe du platine est un catalyseur au ruthénium.